(19) Europäisches Patentamt
European Patent Office
Office européen des brevets

(11) Publication number: 0 409 297 A2

(12) # EUROPEAN PATENT APPLICATION

(21) Application number: 90201586.6

(22) Date of filing: 19.06.90

(51) Int. Cl.5: C07J 21/00, C07J 41/00, C07J 43/00, A61K 31/58

(30) Priority: 20.07.89 GB 8916636

(43) Date of publication of application: 23.01.91 Bulletin 91/04

(84) Designated Contracting States: DE GB IT

(71) Applicant: FARMITALIA CARLO ERBA S.r.L.
Via Carlo Imbonati 24
I-20159 Milano(IT)

(72) Inventor: Bandiera, Tiziano
Corso Vittorio Emanuele,44
I-27025 Gambolo'(Pavia)(IT)
Inventor: Brambilla, Enzo
Via Togliatti, 41b
I-22066 Mariano Comense (Como)(IT)
Inventor: Ferrari, Patrizia
Via Verde, 30
I-21100 Varese(IT)
Inventor: Gobbini, Mauro
Via Isonzo, 14
I-21018 Sesto Calende (Varese)(IT)
Inventor: Mantegani, Sergio
Via Carlo Pisacane, 57
I-20129 Milano(IT)
Inventor: Quadri, Maria Luisa
Via Carlo Porta, 11
Cernusco sul Naviglio (Milano)(IT)
Inventor: Traquandi, Gabriella
Via Cilea, 106
I-20151 Milano(IT)

(74) Representative: Ferrario, Vittorino
c/o Farmitalia Carlo Erba S.r.l. Via Carlo Imbonati 24
I-20159 Milano(IT)

(54) **Steroid ethers.**

(57) Compounds of the formula I

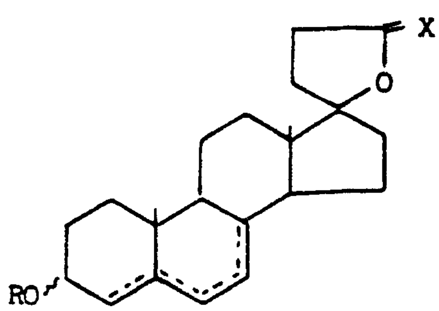

I

wherein X = H,H or O; $R=(CH_2)_nNR_1R_2$, n=2,3; $R_1$ and $R_2$ = $C_1$-$C_4$ alkyl or heterocycle ring when taken together with the nitrogen atom, aminosugar,

• • • • •

EP 0 409 297 A2

means single or double bond and their pharmaceutically acceptable salts are useful as anti-hypertensive agents.
The preparation and use as well as pharmaceutical compositions containing them are also described.

## STEROID ETHERS

The invention relates to steroid ethers, to a process for their preparation and to pharmaceutical compositions containing them.

The invention provides steroid ethers having the formula (I):

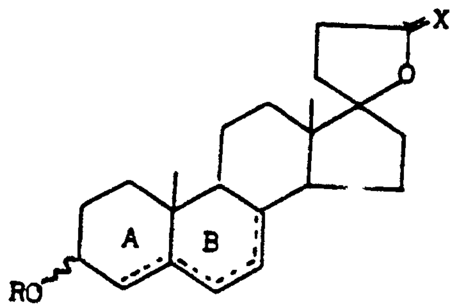

I

wherein X represents two hydrogen atoms or an oxygen atom; R represents

(i) an aminoalkyl residue of the formula $-(CH_2)_n-NR_1R_2$ wherein n represents 2 or 3 and $R_1$ and $R_2$ independently represent a $C_1$-$C_4$ alkyl group or, taken together with the nitrogen atom to which are linked, form a heterocycle residue which contains one or two heteroatoms selected from oxygen and nitrogen and which is optionally substituted by a $C_1$-$C_4$ alkyl group or

(ii) a sugar residue -W wherein W is an amino- or alkylamino-deoxy sugar, an amino- or alkyalmine-dideoxy sugar or an amino- or alkylamino-trideoxy sugar of the D or L series; and the dotted lines mean that the fused rings A and B represent one of the following structures:

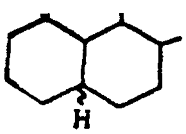 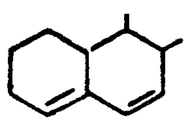 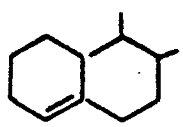 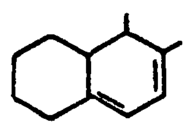 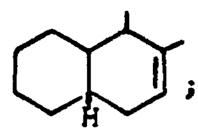 ;

and the pharmaceutically acceptable salts thereof.

Typically the sugar is a $C_5$ or $C_6$ monosaccharide. The alkyl group is generally a $C_1$-$C_4$ alkyl group. A sugar which R may represent is for example

2-amino or 2-alkylamino-2-deoxyhexopyranosyl,

3-amino or 3-alkylamino-3,6-dideoxyhexopyranosyl, or

3-amino or 3-alkylamino-2,3,5-trideoxyhexopyranosyl.

A $C_1$-$C_1$ alkyl group includes methyl, ethyl, i-propyl and n-propyl groups. Preferably R is dimethylaminopropyl. The heterocycle residue which $R_1$ and $R_2$ may represent with the nitrogen atom to which they are linked is preferably of the formula

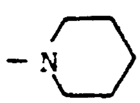 , 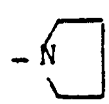 , 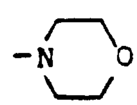 or 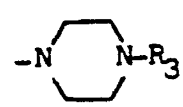

wherein $R_3$ represents a $C_1$-$C_4$ alkyl.

The wavy lines in the formulae indicate that the substituents or the hydrogen atoms may be above or under the plane of the ring system, providing optically active isomer forms having different absolute configurations.

"Pharmaceutically acceptable salts" refers to those salts which retain the biological effectiveness and properties of the free bases and which are not biologically or otherwise undesirable. Such salts are formed with inorganic acids such as hydrochloric, hydrobromic, sulfuric, nitric or phosphoric acid and organic acids such as acetic, propionic, glycolic, pyruvic, oxalic, malic, malonic, succinic, maleic, fumaric, tartaric, citric, benzoic, cinnamic, mandelic, methanesulfonic, ethanesulfonic, p-toluene sulfonic or salicyclic acid.

The invention further provides a process for the preparation of the steroid ethers of the formula (I) or

pharmaceutically acceptable salts thereof, which process comprises condensing a compound having the formula II:

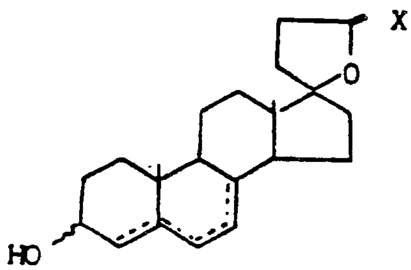

II

wherein X and the dotted lines are as above defined, with an amino alkyl halide of the formula (III) or a sugar derivative of the formula (IV):

$A-(CH_2)_n-NR_1R_2$ (III) A-W (IV)

wherein n, $R_1$, $R_2$ and W are as above defined, and A represents a halogen atom; and, if desired, converting the resulting steroid ether of formula (I) into a pharmaceutically acceptable salt thereof.

The condensation process is desirably carried out in a suitable organic solvent such as tetrahydrofuran, dioxane or dimethylformamide, in the presence of sodium hydride, at a temperature of from 70° to 130°C. Preferably, A represents a chlorine or bromine atom. After the usual work-up the ethers (I) are isolated as such or as an easily crystallizable salt, such as the oxalate.

The steroid derivatives of formula (II), the amino alkylhalides of the formula (III) and the sugar derivatives of the formula (IV) used as starting materials are well known compounds or they may be prepared with procedures familiar to those skilled in the art.

The steroid ethers according to the invention and their pharmaceutically acceptable salts are capable of inhibiting specific ouabain binding without inhibiting $Na^+$, $K^+$-ATPase activity and thus they may be useful agents for the treatment of hypertension.

The compounds of the invention can be administered in a variety of dosage forms, e.g. orally, in the form of tablets, capsules, sugar or film coated tablets, liquid solutions or suspensions; rectally, in the form of suppositories; parenterally, e.g. intramuscularly, or by intravenous injection of infusion; or topically. The dosage depends on the age, weight, conditions of the patient and administration route.

The invention includes pharmaceutical compositions comprising a compound of the invention in association with a pharmaceutically acceptable excipient (which can be a carrier or diluent). The pharmaceutical compositions containing the compounds of the invention are usually prepared following conventional methods and are administered in a pharmaceutically suitable form.

For example, the solid oral forms may contain, together with the active compound, diluents, e.g. lactose, dextrose, saccharose, cellulose, corn starch or potato starch; lubricants, e.g. silica, talc, stearic acid, magnesium or calcium stearate; and/or polyethylene glycols; binding agents, e.g. starches, arabic gums, gelatin, methylcellulose, carboxymethylcellulose or polyvinyl pyrrolidone; disaggregating agents, e.g. a starch, alginic acid, alginates or sodium starch glycolate; effervescing mixtures; dyestuff; sweeteners; wetting agents, such as lecithin, polysorbates, laurysulphates; and, in general, non-toxic and pharmacologically inactive substances used in pharmaceutical formulations. These pharmaceutical preparations may be manufactured in known manner, for example, by means of mixing, granulating, tabletting, sugar-coating, or film-coating processes.

The liquid dispersions for oral administration may be e.g. syrups, emulsions and suspensions. The syrup may contain as carrier, for example, saccharose or saccharose with glycerine and/or manitol and/or sorbitol. The suspensions and the emulsions may contain as carrier, for example, a natural gum, agar, sodium alginate, pectin, methylcellulose, carboxymethylcellulose, or polyvinyl alcohol.

The suspensions or solutions for intramuscular injections may contain, together with the active compound, a pharmaceutically acceptable carrier, e.g. sterile water, olive oil, ethyloleate, glycols, e.g. propylene glycol, and, if desired, a suitable amount of lidocaine hydrochloride.

The solutions for intravenous injections or infusion may contain as carrier, for example, sterile water or, preferably, they may be in the form of sterile, aqueous, isotonic saline solutions.

The suppositories may contain together with the active compound a pharmaceutically acceptable carrier, e.g. cocoa-butter, polyethylene glycol, a polyoxyethylene sorbitan fatty acid ester surfactant or lecithin. Compositions for topical application such as, creams, lotions or pastes, may be prepared by admixing the active ingredient, with a conventional oleaginous or emulsifying excipient.

The following assays were carried out on the compounds of the invention.

"In vitro" assays to test the ability of steroids of formula I to displace specific ouabain binding to the ($Na^+$-$K^+$)-ATPase receptors without inhibiting the ($Na^+$-$K^+$)-ATPase enzymatic activity.

Radiochemical assay:

A microsomial fraction enriched in ($Na^+$-$K^+$)-ATPase was prepared from dog kidney outer medulla, according to Jørgensen (BBA 356: 36-52, 1974).

The partially purified enzyme (0.5µg of protein) was incubated in 3 mM $MgCl_2$, 3 mM EGTA, 80 mM Hepes buffer (pH 7.4) and 2 mM $y^2$ - $P^{32}$ - ATP, final volume 110 µ1, at 37°C for 15 minutes with increasing concentrations of ouabain (as reference compound) or steroid ethers.

The reaction was stopped by the addition of 0.1 mM of cold perchloric acid (10% final concentration) and 0.5 ml of charcoal suspension (20% w/v). The suspension was centrifuged and the content of $^{32}P$ in the supernatant was measured by liquid scintillation counting. (ref. Mall F. et al.; Biochem. Pharm. 33: N. 1, 47-53, 1984).

The effects of various concentrations of steroid ethers and ouabain were expressed as a percentage of inhibition of the total ($Na^+$-$K^+$)-ATPase activity and $IC_{50}$ values were calculated.

Displacement of ouabain ($H^3$) binding from human red blood cells

The procedure has been described elsewhere (Erdmann E. et al.; Arzneim. Forsh 34(II), no. 10: 1314, 1984).

Washed erythrocytes (about 1-1.8 x $10^9$/ml) were incubated in 130 mM NaCl, 1 mM $MgCl_2$, 10 mM glucose, 10 mM sucrose, 10 mM Tris/HCl buffer (pH 7.4) $2x10^{-9}$M $^3H$ ouabain and increasing concentration of the unlabelled steroid ethers at 37°C for 5 hours. Bound ouabain was quantitated by a rapid filtration technique (Whatman GF/C glass filter membranes) to separate free from membrane-bound ouabain. 'Whatman' is a Trade Mark. The radioactivity in the filters was determined by liquid scintillation counting. Non specific binding was defined as the binding in the presence of $10^{-3}$M unlabelled ouabain.

The dissociation constant ($K_D$ value) was calculated from the concentration of unlabelled steroid ethers which inhibit $^3H$-ouabain binding by 50% at equilibrium, by the method of Erdmann et al. (Schmiedeberg's Arch. Pharmacol. 283:335, 1973).

Inhibition of $Na^+$efflux mediated by the ($Na^+$-$K^+$)-ATPase in human red blood cells

The procedure has been described elsewhere (Garay et al., Biochem. Pharmacol. 33:2013-2020, 1984). Washed red blood cells were suspended to a hematocrit of 20-25% in 74 mM $MgCl_2$, 2 mM KCl, 84 mM sucrose, 10 mM MOPS/Tris buffer (pH 7.4 at 37°C) and 10 mM glucose.

Red cell suspensions were added in the cold to tubes containing $Mg^{++}$ sucrose-$K^+$ medium with increasing concentration of ouabain and fixed concentrations of steroid ethers. The tubes were incubated at 37°C and aliquots of the suspensions were transferred to the cold and spun down at different times (0 - 10 - 20 - 30 minutes). External $Na^+$ concentrations were measured in the supernatants by atomic absorption. A kinetic analysis of the inhibition of ouabain sensitive $Na^+$ efflux as a function of different steroid ether concentrations was done and the $IC_{50}$ for each compound was calculated.

'In vivo' assays to test the hypotensive activities of steroid ethers of formula I

Indirect measurements of systolic blood pressure was carried out in groups of 4 spontaneously hypertensive rats (SHR, Kyoto), 8 to 10 weeks of age, supplied by Charles Rives, Italy. The animals were maintained in an environment of 36°C for 10 to 15 minutes to allow pulse pressure to be recorded and then systolic blood pressure and heart rate were measured by the indirect tail cuff method using a W+W, BP recorder, model 8005. The compounds were given orally, suspended in 5% arabic gum, once a day for 4 consecutive days and measurements were carried out before beginning the treatment and 1 and 5 hours

after dosing in both the first and fourth day of treatment. Control animals received the vehicle only (0.2 ml/100 g body weight). Drug induced changes in systolic blood pressure were calculated as differences from the pretreatment values.

The following Examples illustrate the invention.

Example 1

**3β-dimethylaminopropoxy-17α-pregna-4,6-diene-21,17-carbolactone**

To a solution of 0.59 g of 3β-hydroxy-17α-pregna-4,6 -diene-21,17-carbolactone (J.S. Baran, J. Med. Chem. 6 , 329, 1963) in 40 ml of dry tetrahydrofuran, under nitrogen, 0.51 g of sodium hydride (55-60% in mineral oil) were added and the resulting suspension was heated at reflux for an hour. 1.78g of 3-dimethylaminopropyl chloride were added dropwise and the reaction mixture was refluxed for 24 hours. 0.70g of glacial acetic acid in 5 ml of wet tetrahydrofuran were added dropwise, the solvent was evaporated and the residue was taken into ethyl acetate and washed with water until neutrality of the aqueous layer. The organic solution was dried over anhydrous sodium sulphate and evaporated. The product was isolated by column chromatography ($SiO_2$) using ethylene chloride-ethanol 9:1 as the eluant; amorphous solid. FD-MS : m/e 427 ($M^+$). Rf = 0.10 ($SiO_2$ plates, methylene chloride-methanol 9:1); Rf = 0.36 ($SiO_2$, plates, methylene chloride -methanol-30% ammonia solution 90:9:1). Detection: UV lamp (254 nm) or spraying with 2% cerium (IV) sulphate in 2N sulphuric acid and heating.

Example 2

**3α-dimethylaminopropoxy-17α-pregna-4.6-diene-21,17-carbolactone.**

To a solution of 3β-hydroxy-17α-pregna-4,6-diene-21,17 -carbolactone in 120 ml of dry tetrahydrofuran were added 3.07 g of triphenyl phosphine and 0.54 g of formic acid. Under stirring, 2.03 g of diethyl azodicarboxylate in 40 ml of dry tetrahydrofuran were added dropwise. After one hour the solvent was evaporated and the residue was filtered through 40 g of $SiO_2$, using ethyl acetate-cyclohexane (1:1) as the eluant. The first fractions were collected and evaporated. The residue was hydrolyzed in dioxane with 5% sodium hydrogen carbonate solution. After the usual work-up the product was purified by column chromatography to give white crystals of 3α-hydroxy-17α-pregna-4,6-diene-21,17-carbolactone melting at 159-162° C.

3α-hydroxy-17α-pregna-4,6-diene-21,17-carbolactone was reacted with 3-dimethylaminopropyl chloride as described in example 1 to give 3α-dimethylaminopropoxy-17α-pregna-4,6-diene-21,17-carbolactone as a colourless oil. FD-MS : m/e 427 ($M^+$). Rf = 0.10 ($SiO_2$ plates, methylene chloride-methanol 9:1); Rf = 0.35 ($SiO_2$ plates, methylene chloride-methanol-30% ammonia solution 90:9:1). netection: UV 1amp (254 nm) or spraying with 2% cerium (IV) sulphate in 2N sulphuric acid and heating.

Example 3

**3β-dimethylaminopropoxy-androst-4,6-diene-17(R)-spiro-2′-oxolane.**

3β-hydroxy-androst-4,6-diene-17(R)-spiro-2′-oxolane (E.G. Arth et al., U.S. Patent 3,753,979) was reacted with 3-dimethylaminopropyl chloride as described in example 1 to give 3β-dimethylaminopropoxy-androst-4,6-diene-17(R)-spiro-2′-oxolane as colourless oil. FD-MS : m/e 413 ($M^+$). Rf = 0.11 ($SiO_2$ plates, methylene chloride-metanol 9:1). Rf = 0.30 ($SiO_2$ plates, methylene chloride-methanol-30% ammonia solution 90:9:1). section: UV lamp (254 nm) or spraying with 2% cerium (IV) sulphate in 2N sulphuric acid and heating.

Example 4

**3β-dimethylaminopropoxy-17α-pregna-5,7-diene-21,17-carbolactone**

3β-hydroxy-17α-pregna-5,7-diene-21,17-carbolactone (A.E. Brown, U.S. Patent 3,886,146) was reacted with 3-dimethylaminopropyl chloride as described in example 1 to give 38-dimethylaminopropoxy-17α--pregna-5,7-diene-21,17-carbolactone as an amorphous solid. FD-MS : m/e 427 ($M^+$). Rf = 0.11 ($SiO_2$ plates, methylene chloride-methanol 9:1); Rf = 0.34 ($SiO_2$ plates, methylene chloride-methanol-30% ammonia solution 90:9:1). netection: UV lamp (254 nm) or spraying with 2% cerium (IV) sulphate in 2N sulphuric acid and heating.

Example 5

**3β-dimethylaminopropoxy-androst-5,7-diene-17(R)-spiro-2′-oxolane**

3β-hydroxy-androst-5,7-diene-17(R)-spiro-2′-oxolane (A.E. Brown, ibid.) is reacted with 3-dimethylaminopropyl chloride as described in example 1 to give 3β-dimethylaminopropory-androst-5,17-diene-17(R)-spiro-2′-oxolane as an amorphous solid. FD-MS : m/e 413 ($M^+$). Rf = 0.11 ($SiO_2$ plates, methylene chloride-methanol 9:1); Rf = 0.35 ($SiO_2$ plates, methylene chloride-methanol-30% ammonia solution 90:9:1). Detection: UV 1amp (254 nm) or spraying with 2% cerium (IV) sulphate in 2N sulphuric acid.

Example 6

**3β-dimethylaminopropoxy-5α-17α-pregna-7-ene-21,17-carbolactone**

To a solution of 1 g of 3β-hydroxy-17α-pregna-5,7-diene -21,17-carbolactone (A.E. Brown, ibid.) in 100 ml of dioxane, 1 g of Raney-Nickel was added. The reaction mixture was stirred under hydrogen at room temperature for 2 hours. The catalyst was filtered and the solvent was evaporated. The residue was crystallized from ethyl acetate to give 3β-hydroxy-5α,17α-pregna-7-ene-21,17-carbolactone, m.p. 226-228° C.

3β-hydroxy-5α,17α-pregna-7-ene-21,17-carbolactone was reacted with 3 -dimethylaminopropyl chloride as described in example 1 to give 3β-dimethylamino propaxy-5α,17α-pregna-7-ene-21,17-carbolactone as a white solid, m.p. 205-210° C(dec.). FD-MS : m/e 429 ($M^+$). Rf = 0.36 ($SiO_2$ plates, methylene chloride-methanol-30% ammonia solution 90:9:1). Detection: 2% cerium (IV) sulphate in 2N sulphuric acid and heating.

Example 7

**3β-dimethylaminopropoxy-5α-androst-7-ene-17(R)-spiro-2′oxolane**

3β-hydroxy-androst-5,7-diene-17(R)-spiro-2′-oxolane (A.E. Brown, ibid.) was reduced to 3β-hydroxy-5α-androst-7-ene-17(R)-spiro-2′-oxolane m.p. 164-165° C, as reported in example 6.

3β-hydroxy-5α-androst-7-ene-17(R)-spiro-2′-oxolane was reacted with 3 -dimethylamino propyl chloride as described in example 1 to give 3β-dimethylaminopropoxy-5α-androst-7-ene-17(R)-spiro-2′-oxolane as an amorphous solid. FD-MS : m/e 415 ($M^+$). Rf = 0.10 ($SiO_2$ plates, methylene chloride-methanol 9:1); Rf = 0.34 (SiO, plates, methylene chloride-methanol- 30% ammonia solution 90:9:1). Detection: 2% cerium (IV) sulphate in 2N sulphuric acid and heating.

Example 8

**3α-dimethylaminopropyloxy-5β-,17α-pregnan-21,17-carbolactone**

Operating as in Example 1, but employing 3α-hydroxy-5β,17α-pregnan-21,17 carbolactone, the title compound was obtained in 60% yield. m.p. 137°-139° C.

Example 9

3β-(2-(1-pyrrolidino)ethyl)oxy-17α-pregnan-4,6-diene-21,17-carbolactone

Operating as in Example 2, but employing N-(2-chloroethyl) pyrrolidine, the title compound was obtained in 60% yield. m.p. 159°-161° C.

Example 10

**3β-(2-(4-morpholinyl)ethyl)oxy-17α-pregnan-4,6-diene-21,17-carbolactone**

Operating as in Example 1, but employing N-(2-chloroethyl) morpholine, the title compound was obtained in 50% yield. m.p. 173°-175° C.

Example 11

**3β-dimethylaminoethoxy-androsta-4,6-diene-17(R)-spiro-2′-oxolane**

Operating as in Example 1 but using 3-hydroxy-androsta-4,6-diene-17(R)-spiro-2′-oxolane and 2-dimethylamino ethylchloride, the title compound was obtained in 30% yield. m.p. 163°-165° C.

Example 12

**3β-dimethylaminoethoxy-androsta-5,7-diene-17(r)-spiro-2′--oxolane.**

Operating as in Example 1, but using 3β-hydroxy-androsta--5′,7-diene-17(R)-spiro-2′-oxolane and 2-dimethylaminoethylchloride, the title compound was obtained in 35% yield. m.p. 88°-91° C.

Example 13

**3α-dimethylaminopropoxy-17α-pregn-4-ene-21, 17-carbolactone**

Operating as in Example 1, but employing 3α-hydroxy-17α-pregn-4-ene-21,17-carbolactone, the title compound was obtained in 55% yield. m.p. 121°-123° C.

## Claims

1. A steroid ether having the formula (I):

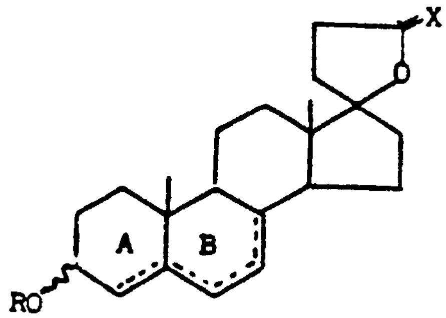

I

wherein x represents two hydrogen atoms or an oxygen atom; R represents

(i) an aminoalkyl residue of the formula $-(CH_2)_n-NR_1R_2$ wherein n represents 2 or 3 and $R_1$ and $R_2$ independently represent a $C_1$-$C_4$ alkyl group or, taken together with the nitrogen atom to which are linked, form a heterocycle residue which contains one or two heteroatoms selected from oxygen and nitrogen and which is optionally substituted by a $C_1$-$C_4$ alkyl group or

(ii) a sugar residue -W wherein W is an amino- or alkylamino-deoxy sugar, an amino- or alkylamino-dideoxy sugar or an amino- or alkylamino-trideoxy sugar of the D or L series; and the dotted lines mean that the fused rings A and B represent one of the following structures:

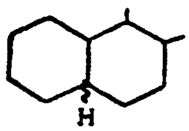 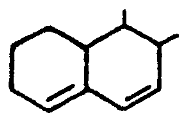 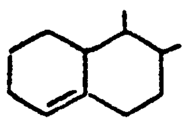 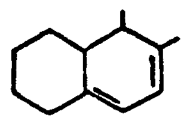 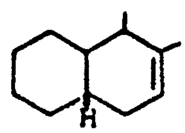 ;

and the pharmaceutically acceptable salts thereof.

2. A compound according to claim 1, wherein R is dimethylaminopropyl.

3. A compound according to claim 1 wherein $R_1$ and $R_2$, taken together with the nitrogen atom to which they are linked, form a heterocycle of the formula:

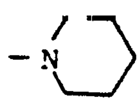 , 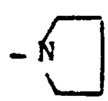 , 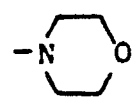 or 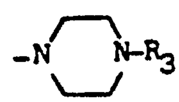

wherein $R_3$ represents $C_1$-$C_4$ alkyl.

4. A compound according to claim 1, which is selected from:

3β-dimethylaminopropoxy-17α-pregna-4,6-diene-21,17-carbolactone,
3α-dimethylaminopropoxy-17α-pregna4 ,6-diene-21,17-carbolactone,
3β-dimethylaminopropoxy-androst-4,6-diene-17(R)-spiro-2′-oxolane,
3β-dimethylaminopropoxy-17α-pregna-5,7-diene-21,17-carbolactone,
3β-dimethylaminopropoxy-androst-5,7-diene-17(R)-spiro- 2′-oxolane,
3β-dimethylaminopropoxy-5α, 17 -pregna-7-ene-21,17-carbolactone, and
3β-dimethylaminopropoxy-5α-androst-7-ene-17(R)-spiro-2′-oxolane.

5. A process for the preparation of a steroid ether of formula (I) as defined in claim 1 or a pharmaceutically acceptable salt thereof, which process comprises condensing a compound of formula (II):

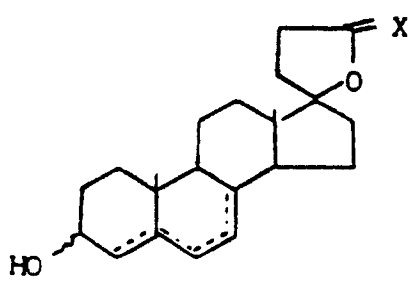

II

wherein X and the dotted lines are as defined in claim 1, with an amino alkyl halide of the formula (III) or a sugar derivative of the formula (IV):

$A-(CH_2)_n-NR_1R_2$ (III) A-W (IV)

wherein n, $R_1$, $R_2$ and in are as defined in claim 1 and A represents a halogen atom; and if desired converting the resulting steroid ether of formula (I) into a pharmaceutically acceptable salt thereof.

6. A process according to claim 5 wherein the condensation is carried out in an organic solvent, in the presence of sodium hydride, at a temperature of from 70° to 130° C.

7. A process according to claim 5 or 6, wherein A represents a chlorine or bromine atom.

8. A pharmaceutical composition comprising a pharmaceutically acceptable carrier or diluent and, as active ingredient, a steroid ether of formula (I) or pharmaceutically acceptable salt thereof as claimed in any one of claims 1 to 4 or which has been produced by a process as claimed in any one of claims 5 to 7.

9. A steroid ether of formula (I) as defined in claim 1 or pharmaceutically acceptable salt thereof for use in a method of treatment of the human or animal body by therapy.

10. A compound according to claim 9 for use in the treatment of hypertension.